# EUROPEAN PATENT APPLICATION

(11) **EP 4 527 366 A1**
(43) Date of publication of application: **26.03.2025**
(21) Application number: 23831471.0
(22) Date of filing: 27.06.2023
(51) Int. Cl.: A61F 13/42, A61F 13/494, A61F 13/511, A61F 13/532, A61F 13/533

(54) **DIAPER**

(30) Priority: 27.06.2022 JP 2022102862; 17.05.2023 WO PCT/JP2023/018404
(71) Applicant: Unicharm Corporation, Ehime 799-0111 (JP)
(72) Inventor: TSUJII, Maki, Kanonji-shi, Kagawa 769-1602 (JP)
(74) Representative: Dolleymores
(86) International application number: PCT/JP2023/023863
(87) International publication number: WO 2024/005031

(57) **Abstract**

A diaper of an aspect includes: an absorbent main body including a top sheet disposed on a skin surface side of a wearer, a back sheet disposed on a non-skin surface side on the opposite side to the skin surface side, and an absorbent core disposed between the top sheet and the back sheet; and a pair of fastening tabs extending out from the absorbent main body to the outside in a lateral direction. The absorbent main body is formed with a guide mark for guiding the position where the buttocks of the wearer are to be placed so as to be visible from the skin surface side, and the absorbent core is at least partially constricted inward in the lateral direction at a position on the ventral side relative to the guide mark.

## Description

### Technical Field

The present disclosure relates to a diaper.

### Background Art

A common tape-type diaper includes an absorbent main body including a top sheet, a back sheet, and an absorbent core disposed between the top sheet and the back sheet, and a pair of fastening tabs extending out to the outside of the absorbent main body. When putting on this type of diaper, the wearer is laid on his/her back on the spread diaper, the diaper is pulled up to the ventral side through the crotch of the wearer, and the pair of fastening tabs are locked to a portion to be locked disposed on the ventral side of the absorbent main body.

When putting the diaper on the wearer, if the position where the wearer is laid down is wrong, the positions of both ends in the lengthwise direction of the diaper are not aligned in the up-down direction of the body, and consequently the pair of fastening tabs cannot be locked to the portion to be locked in the correct position. If the position of the diaper is displaced in the lengthwise direction, gaps are likely to be formed around the legs or the waist of the wearer, and urine leakage is caused. In order to solve such an issue, Patent Literature 1 below discloses a diaper in which a mark providing guidance to the position of the buttocks of the wearer is displayed on the top sheet.

### Citation List

### Patent Literature

Patent Literature 1: Japanese Unexamined Patent Publication No. 2014-230716

### Summary of Invention

### Technical Problem

In a diaper like the above, when pulling up the diaper to the ventral side through the crotch of the wearer, there is a case where the absorbent core of the diaper is caught around the legs of the wearer and the diaper cannot be sufficiently pulled up to the ventral side of the wearer. If the pulling-up of the diaper is insufficient, the position of the diaper may be displaced in the lengthwise direction even when the wearer is laid down in the correct position.

Thus, an object of the present disclosure is to suppress displacement of a diaper.

### Solution to Problem

In an aspect, there is provided a diaper extending in a longitudinal direction connecting from the ventral side to the dorsal side of the wearer and in a lateral direction perpendicular to the longitudinal direction. This diaper includes an absorbent main body including a top sheet disposed on the skin surface side of the wearer, a back sheet disposed on the non-skin surface side on the opposite side to the skin surface side, and an absorbent core disposed between the top sheet and the back sheet, and a pair of fastening tabs extending out from the absorbent main body to the outside in the lateral direction. The absorbent main body is formed with a guide mark for guiding a position where buttocks of the wearer are to be placed so as to be visible from the skin surface side, and the absorbent core is at least partially constricted inward in the lateral direction at a position on the ventral side relative to the guide mark.

In the diaper of the present aspect, since the absorbent core is at least partially constricted inward on the ventral side relative to the guide mark, when pulling up the diaper to the ventral side through the crotch of the wearer, the absorbent core is less likely to interfere with the legs of the wearer. Therefore, the diaper can be sufficiently pulled up to the ventral side, and as a result displacement of the diaper can be suppressed.

In an embodiment, the absorbent core may have a pair of channel portions formed symmetrically with respect to a longitudinal center line passing through the center in the lateral direction of the absorbent main body and having a lower basis weight than the other portions of the absorbent core, and at least parts of the pair of channel portions may be disposed on the ventral side relative to the guide mark. The rigidity of the pair of channel portions is lower than that of the other portions of the absorbent core; thus, when the absorbent core comes into contact with the legs of the wearer, the absorbent core is easily bent from the pair of channel portions as starting points. Therefore, when pulling up the diaper to the ventral side through the crotch of the wearer, the absorbent core is less likely to interfere with the legs of the wearer by being bent. As a result, the diaper can be sufficiently pulled up to the ventral side, and displacement of the diaper is suppressed.

In an embodiment, the absorbent core may include a constricted region having a narrower width in the lateral direction than an end portion on the dorsal side of the absorbent core, and as viewed from the skin surface side, the guide mark may be disposed in a position overlapping with the constricted region. The area of the portion of the constricted region on the ventral side relative to the guide mark of the constricted region may be larger than the area of the portion the constricted region on the dorsal side relative to the guide mark of the constricted region. In this embodiment, when pulling up the diaper to the ventral side through the crotch of the wearer, the absorbent core is less likely to interfere with the legs of the wearer.

In an embodiment, the absorbent main body may have an indicator extending in the longitudinal direction and configured to change color by reacting with a body fluid of the wearer, and the guide mark may be disposed on the dorsal side relative to the end on the dorsal side of the indicator. By the indicator being disposed on the ventral side relative to the guide mark, the indicator easily comes into contact with urine of the wearer, and allows the user to recognize urination.

In an embodiment, the top sheet may include a center sheet covering the absorbent core and a pair of side sheets covering both side portions of the center sheet in the lateral direction, each of the pair of side sheets may include a dorsal-side fixed portion fixed to the center sheet on the dorsal side relative to a transverse center line passing through the center in the longitudinal direction of the absorbent main body, and in the longitudinal direction, the guide mark may be disposed on the ventral side relative to the midpoint between the end on the ventral side of the dorsal-side fixed portion and the transverse center line. By the guide mark being disposed on the ventral side relative to the midpoint between the end on the ventral side of the dorsal-side fixed portion and the transverse center line, the buttocks of the wearer can be guided to the correct position.

In an embodiment, the guide mark may have a shape imitating the shape of the buttocks. By forming the guide mark in a shape imitating the shape of the buttocks, the user can be urged to place the buttocks of the wearer in accordance with the guide mark.

In an embodiment, the guide mark may include two curved portions convex on the ventral side. By inclusion of two curved portions convex on the ventral side, the position and direction in which the buttocks are to be placed can reliably be recognized by the user.

In an embodiment, the guide mark may include a coupling portion in which one ends of the two curved portions are coupled to each other, and the coupling portion may be placed on a longitudinal center line passing through the center in the lateral direction of the absorbent main body. By placing a coupling portion on the longitudinal center line, urging can be made to place the buttocks in accordance with the center in the width direction. The other ends of the two curved portions may be disposed on the dorsal side relative to the coupling portion.

In an embodiment, when a portion most protruding on the ventral side of the two curved portions is defined as a lowest point, a first angle formed by an imaginary line connecting the lowest point and one end of the two curved portions with respect to a reference line parallel to the lateral direction is larger than a second angle formed by an imaginary line connecting the lowest point and the other end of the two curved portions with respect to the reference line. By setting the first angle larger than the second angle, the guide mark can be formed in a shape imitating the buttocks.

In an embodiment, the line width of the two curved portions may be 0.3% or more and 3.5% or less with respect to the total length of the absorbent main body in the longitudinal direction. If the line width of the two curved portions is too small, it is difficult for the user to recognize the guide mark, and if the line width of the two curved portions is too large, it is difficult to place the buttocks in the correct position. By setting the line width of the two curved portions to 0.3% or more and 3.5% or less with respect to the total length of the absorbent main body, displacement of the position of the buttocks can be suppressed while the guide mark is made easily recognizable. The width of the guide mark in the lateral direction may be larger than the width of the guide mark in the longitudinal direction.

In an embodiment, the absorbent main body may include a pair of standing leakage-preventing gathers and placed on the outside in the lateral direction of the absorbent core; when the pair of leakage-preventing gathers are in a standing state, the other ends of the two curved portions of the guide mark may be exposed, and when the pair of leakage-preventing gathers are in a non-standing state, the other ends of the two curved portions of the guide mark may be covered with the pair of leakage-preventing gathers. In this embodiment, the guide mark can be visible when putting on the diaper, and therefore guidance to the position where the buttocks are to be placed can be appropriately provided.

In an embodiment, the pair of channel portions may extend from a position on the ventral side relative to the guide mark to a position on the dorsal side relative to the guide mark, and the length of the portion of the pair of channel portions on the ventral side relative to the guide mark may be longer than a length of a portion of the pair of channel portions on the dorsal side relative to the guide mark. By increasing the length of the portion on the ventral side relative to the guide mark of each of the pair of channel portions, the absorbent core is easily bent on the ventral side relative to the guide mark, and is less likely to interfere with the legs of the wearer.

In an embodiment, the width of the guide mark in the lateral direction may be larger than the distance between the pair of channel portions in the lateral direction. In this embodiment, the absorbent core is easily bent along the cleft of the buttocks of the wearer, and is less likely to interfere with the legs of the wearer. The ends on the dorsal side of the pair of channel portions may be placed on the ventral side relative to the guide mark.

In an embodiment, the absorbent core may include an embossed portion subjected to unevenness processing, and as viewed from the skin surface side, the guide mark may be disposed at a position overlapping with the embossed portion. Wrinkles are less likely to occur in the embossed portion, and therefore the guide mark is made easily visually recognizable by being disposed in a position overlapping with the embossed portion.

In an embodiment, the guide mark may be printed on a sheet material disposed between the top sheet and the absorbent core. The sheet material may be tissue containing pulp. By printing the guide mark on a sheet material placed between the top sheet and the absorbent core, the ink is less likely to move to the buttocks of the wearer. The guide mark may be printed on the surface on the non-skin surface side of the sheet material. Thereby, the ink can more reliably be prevented from moving to the buttocks of the wearer.

In an embodiment, at least one of the surface on the skin surface side and the surface on the non-skin surface side of the sheet material may be bonded to the top sheet or the absorbent core with a hot melt adhesive. By at least one surface of the sheet material being bonded to the top sheet or the absorbent core, a situation where the sheet material is rubbed against the top sheet or the absorbent core and broken is suppressed.

In an embodiment, when the side sheets are folded on the inside in the lateral direction, at least part of the guide mark may be formed in a position visually recognizable from the skin surface side. By virtue of the fact that the guide mark can be visually recognized in a state where the side sheets are folded, the presence of the guide mark can be easily recognized by the user.

In another aspect, there is provided a diaper extending in a longitudinal direction connecting from the ventral side to the dorsal side of the wearer and in a lateral direction perpendicular to the longitudinal direction. This diaper includes an absorbent main body including a top sheet disposed on the skin surface side of the wearer, a back sheet disposed on the non-skin surface side of the wearer, and an absorbent core disposed between the top sheet and the back sheet, and a pair of fastening tabs extending out from the absorbent main body to the outside in the lateral direction. The absorbent main body is formed with a guide mark for guiding the position where the buttocks of the wearer are to be placed so as to be visible from the skin surface side, the absorbent core has a pair of channel portions formed symmetrically with respect to a longitudinal center line passing through the center in the lateral direction of the absorbent main body and having a lower basis weight than the other portions of the absorbent core, and at least parts of the pair of channel portions are disposed on the ventral side relative to the guide mark.

In the diaper of the present aspect, a pair of channel portions having a low basis weight are formed on the ventral side relative to the guide mark. The rigidity of the pair of channel portions is lower than that of the other portions of the absorbent core; thus, when the absorbent core comes into contact with the legs of the wearer, the absorbent core is easily bent from the pair of channel portions as starting points. Therefore, when pulling up the diaper to the ventral side through the crotch of the wearer, the absorbent core is bent and is less likely to interfere with the legs of the wearer. As a result, the diaper can be sufficiently pulled up to the ventral side, and displacement of the diaper is suppressed.

In an embodiment, the absorbent core may be at least partially constricted inward in the lateral direction at a position on the ventral side relative to the guide mark. Since the absorbent core is at least partially constricted at a position on the ventral side relative to the guide mark, when pulling up the diaper to the ventral side through the crotch of the wearer, the absorbent core is less likely to interfere with the legs of the wearer. Therefore, the diaper can be sufficiently pulled up to the ventral side, and displacement of the diaper can be suppressed.

### Advantageous Effects of Invention

According to various aspects and embodiments of the present invention, displacement of the diaper can be suppressed.

### Brief Description of Drawings

FIG. 1 is a plan view of a diaper according to an embodiment as viewed from a skin surface side.
FIG. 2 is a plan view of the diaper shown in FIG. 1 as viewed from a non-skin surface side.
FIG. 3 is a cross-sectional view taken along line III-III shown in FIG. 1.
FIG. 4 is an enlarged view of an area around a guide mark.
FIG. 5 is a plan view of a diaper according to another embodiment.
FIG. 6 is a plan view of a diaper according to still another embodiment.
FIG. 7 is a plan view of a diaper according to still another embodiment.
FIGS. 8(a) to 8(d) are diagrams showing modification examples of the guide mark.
FIGS. 9(a) to 9(d) are diagrams showing modification examples of the guide mark.
FIG. 10 is a diagram showing a modification example of the guide mark.

### Description of Embodiments

Hereinbelow, embodiments of the present disclosure are described with reference to the drawings. In the following description, the same or equivalent elements are denoted by the same reference numerals, and a repeated description is omitted. The dimensional ratios in the drawings do not necessarily coincide with those in the description.

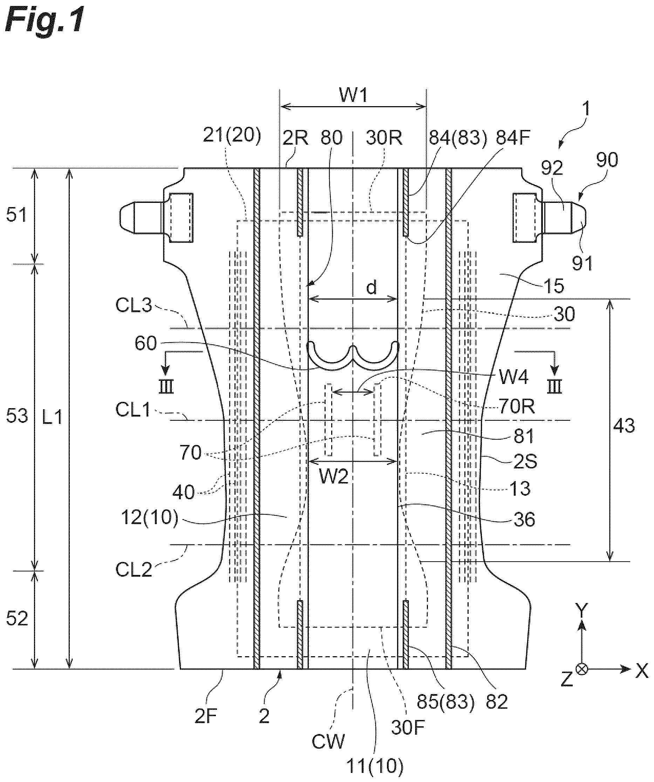

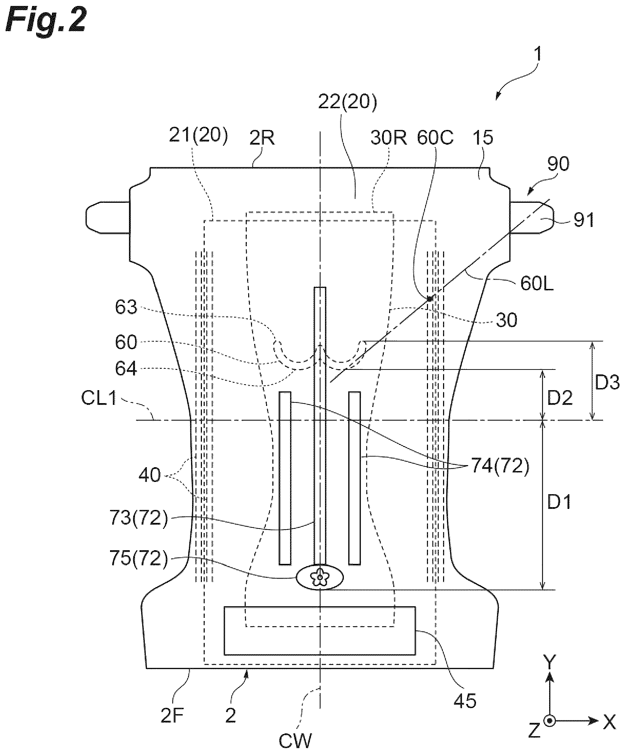

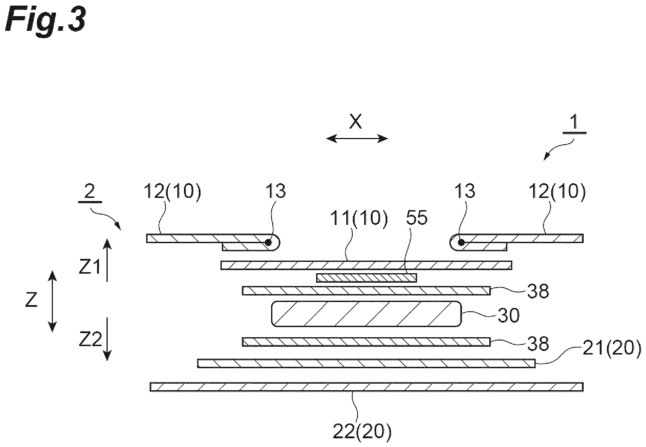

FIG. 1 is a plan view of a diaper 1 according to an embodiment as viewed from a skin surface side. FIG. 2 is a plan view of the diaper 1 as viewed from a non-skin surface side. FIG. 3 is a schematic cross-sectional view of the diaper 1 taken along line III-III shown in FIG. 1. FIGS. 1 and 2 show the diaper 1 in an unfolded state of being spread up to a state where wrinkles are not formed. In the following description, positional relationships in the unfolded state are described unless otherwise stated. In the cross-sectional view of FIG. 3, for convenience of description, the members are shown apart from each other in the thickness direction Z; however, in an actual product, the members are in contact with each other in the thickness direction Z.

The diaper 1 shown in FIGS. 1 to 3 is a tape-type disposable diaper. The diaper 1 may be a child diaper or an adult diaper. In the following description, a person who wears the diaper 1 is referred to as a "wearer", and a person who puts the diaper 1 on the wearer is referred to as a "user". Typically, the wearer is a child or a care receiver, and the user is a parent or a caregiver.

As shown in FIG. 1, the diaper 1 extends in a lateral direction X disposed along the waistline direction of the wearer and in a longitudinal direction Y connecting from the ventral side to the dorsal side of the wearer. The lateral direction X corresponds to the width direction of the diaper 1, and the longitudinal direction Y corresponds to the lengthwise direction of the diaper 1. The lateral direction X and the longitudinal direction Y are directions perpendicular to each other. The thickness direction Z is a direction perpendicular to the lateral direction X and the longitudinal direction Y, and includes a skin surface side Z1 facing the skin surface of the wearer in the wearing state and a non-skin surface side Z2 facing the opposite side to the skin surface of the wearer in the wearing state. The diaper 1 is put on to cover the abdomen, the crotch, and the back of the wearer.

The diaper 1 includes an absorbent main body 2 and a pair of fastening tabs 90. The absorbent main body 2 has a ventral-side edge 2F located on one side in the longitudinal direction Y and a dorsal-side edge 2R located on the other side in the longitudinal direction Y. The ventral-side edge 2F is disposed in the waistline direction of the wearer on the ventral side during wearing, and the dorsal-side edge 2R is disposed in the waistline direction of the wearer on the dorsal side during wearing. The distance in the longitudinal direction Y between the ventral-side edge 2F and the dorsal-side edge 2R is the total length (maximum length) L1 of the absorbent main body 2. In the following description, the ventral-side edge 2F in the longitudinal direction Y is referred to as a ventral side, and the dorsal-side edge 2R in the longitudinal direction Y is referred to as a dorsal side.

The absorbent main body 2 includes a dorsal-side region 51 located on the dorsal side, a ventral-side region 52 located on the ventral side, and an intermediate region 53 located between the dorsal-side region 51 and the ventral-side region 52. The width of the intermediate region 53 in the lateral direction X is smaller than each of the widths of the dorsal-side region 51 and the ventral-side region 52 in the lateral direction X.

In the dorsal-side region 51 of the absorbent main body 2, a pair of flap portions 15 protruding from the absorbent main body 2 to the outside in the lateral direction X are formed. The width of the pair of flap portions 15 in the lateral direction X corresponds to the maximum width of the absorbent main body 2.

The pair of flap portions 15 are individually provided with a pair of fastening tabs 90. The pair of fastening tabs 90 extend out from the side edges 2S of the absorbent main body 2 to the outside in the lateral direction X. Each of the pair of fastening tabs 90 includes a base material sheet 91 joined to the flap portion 15 and a joint portion 92 provided on the base material sheet 91. The joint portion 92 is disposed on the surface on the skin surface side Z1 of the base material sheet 91. The joint portion 92 is, for example, a mechanical fastener, and is configured to be joinable to a target portion 45 formed on the non-skin surface side Z2 of the absorbent main body 2.

As shown in FIG. 2, the target portion 45 is provided on the non-skin surface side Z2 of the ventral-side region 52 of the absorbent main body 2. The target portion 45 has a strip-like shape extending in the lateral direction X. The target portion 45 is disposed on the ventral side relative to the pair of fastening tabs 90, and engages with the pair of fastening tabs 90. Also a configuration in which the absorbent main body 2 does not include the target portion 45 and the joint portions 92 of the pair of fastening tabs 90 are directly joinable to a back sheet 20 of the absorbent main body 2 described later is possible.

As shown in FIGS. 1 to 3, the absorbent main body 2 includes a top sheet 10, a back sheet 20, and an absorbent core 30. The top sheet 10 forms, in the absorbent main body 2, a surface that is brought into contact with the wearer, and is disposed on the skin surface side Z1. The top sheet 10 includes a center sheet 11 disposed at the center in the lateral direction X and covering the absorbent core 30, and a pair of side sheets 12 covering both side portions in the lateral direction X of the center sheet 11. The center sheet 11 is formed of, for example, a liquid-permeable nonwoven fabric that allows urine of the wearer to permeate on the absorbent core 30 side, and the side sheet 12 is formed of, for example, a liquid-impermeable nonwoven fabric.

As shown in FIG. 3, inner portions of the pair of side sheets 12 in the lateral direction X are folded back to the non-skin surface side Z2. A pair of elastic members 13 in a state of being extended in the longitudinal direction Y are disposed individually inside the folded pair of side sheets 12. The pair of side sheets 12 and the pair of elastic members 13 constitute a pair of leakage-preventing gathers 80. The pair of leakage-preventing gathers 80 are gathers for leakage prevention having rising properties, and are disposed on the skin surface side Z1 relative to the absorbent core 30.

As shown in FIG. 1, the pair of leakage-preventing gathers 80 are disposed on the outside in the lateral direction X of the absorbent core 30, and suppresses a situation where urine excreted to the absorbent core 30 leaks in the lateral direction X. Each of the pair of leakage-preventing gathers 80 includes an elastic member 13 extending in the longitudinal direction Y, a rising portion 81 that rises by contraction of the elastic member 13, a lateral fixed unit 82 serving as a rising fulcrum of the rising portion 81 in the lateral direction X, and a longitudinal fixed unit 83 serving as a rising fulcrum of the rising portion 81 in the longitudinal direction Y.

The elastic member 13 extends in the longitudinal direction Y along the inner edge in the lateral direction X of the side sheet 12. The elastic member 13 is formed of, for example, a stretchable material such as rubber or spandex that can contract in the length direction. The elastic members 13 of the pair of leakage-preventing gathers 80 are disposed apart from each other beyond a longitudinal center line CW passing through the center in the lateral direction X of the absorbent main body 2, and are fixed to the side sheets 12 in a state of extending in the longitudinal direction Y. The end on the dorsal side of the elastic member 13 is disposed in the dorsal-side region 51, and the end on the ventral side of the elastic member 13 is disposed in the ventral-side region 52.

The lateral fixed unit 82 is formed substantially in the entire region in the longitudinal direction Y of the absorbent main body 2 in a position on the outside in the lateral direction X of the rising portion 81 of the side sheet 12. That is, the lateral fixed unit 82 is disposed on the outside of the absorbent core 30 in the lateral direction X. The lateral fixed unit 82 is a part of the side sheet 12, and is fixed to the center sheet 11. The lateral fixed unit 82 serves as a rising fulcrum of the rising portion 81 in the lateral direction X.

The longitudinal fixed unit 83 is formed on the inside in the lateral direction X with respect to the lateral fixed unit 82. The longitudinal fixed unit 83 includes a dorsal-side fixed portion 84 disposed in the dorsal-side region 51 and a ventral-side fixed portion 85 disposed in the ventral-side region 52. The dorsal-side fixed portion 84 and the ventral-side fixed portion 85 are parts of the side sheet 12, and are fixed to the center sheet 11. The dorsal-side fixed portion 84 and the ventral-side fixed portion 85 serve as rising fulcrums on the dorsal side and the ventral side of the rising portion 81, respectively.

The rising portion 81 is formed between the dorsal-side fixed portion 84 and the ventral-side fixed portion 85. That is, the rising portion 81 is disposed in a position on the inside relative to the lateral fixed unit 82 in the lateral direction X and on the inside relative to the longitudinal fixed unit 83 in the longitudinal direction Y. The rising portion 81 is a portion of the side sheet 12 not fixed to the center sheet 11. The elastic member 13 is connected to the rising portion 81 in a state of being extended in the longitudinal direction Y. By the elastic members 13 contracting in the longitudinal direction Y, the absorbent main body 2 is deformed in the closing direction, and the rising portions 81 rise from the lateral fixed units 82 as starting points. Urine leakage is prevented by the rising portions 81 thus rising in positions where the absorbent core 30 is sandwiched from the lateral direction X.

The back sheet 20 forms, in the absorbent main body 2, a surface located on the outside during wearing, and is disposed on the non-skin surface side Z2. The back sheet 20 includes a liquid-impermeable back surface film 21 and a back surface nonwoven fabric 22 located on the non-skin surface side Z2 relative to the back surface film 21. The width in the lateral direction X of the back surface film 21 may be formed shorter than the width in the lateral direction X of the back surface nonwoven fabric 22, and the back surface nonwoven fabric 22 may extend out to both sides in the lateral direction X relative to the back surface film 21. The back surface nonwoven fabric 22 forms the outermost layer of the diaper 1.

The absorbent core 30 is disposed between the top sheet 10 and the back sheet 20. The absorbent core 30 contains, for example, pulp fiber and a polymer absorbent (SAP), which have water absorbency, and absorbs urine of the wearer. As shown in FIG. 3, the upper surface and the lower surface of the absorbent core 30 may be covered with core wrap 38. The absorbent core 30 is disposed substantially at the center in the lateral direction X of the absorbent main body 2.

The absorbent core 30 has an edge 30R on the dorsal side and an edge 30F on the ventral side. The edge 30R on the dorsal side of the absorbent core 30 has a width W1 in the lateral direction X. The width W1 is the maximum width of the absorbent core 30. The width W1 of the edge 30R on the dorsal side is larger than the distance d between the pair of leakage-preventing gathers 80 in the non-standing state. The edge 30F on the ventral side of the absorbent core 30 has, for example, the same width as the edge 30R on the dorsal side. A constricted region 43 in which the width of the absorbent core 30 is narrowed is formed between the edge 30F on the ventral side and the edge 30R on the dorsal side. The constricted region 43 is, for example, a region of the absorbent core 30 in which the width in the lateral direction X is narrowed by 10% or more with respect to the width W1 of the edge 30F on the ventral side and the edge 30R on the dorsal side.

The constricted region 43 includes a narrowed portion 36 in which the width is most narrowed in the lateral direction X. The width W2 of the narrowed portion 36 is smaller than the width W1 of the edge 30R on the dorsal side of the absorbent core 30. The width of the constricted region 43 is continuously narrowed from the edge 30R on the dorsal side toward the narrowed portion 36. Similarly, the width of the constricted region 43 is continuously narrowed from the edge 30F on the ventral side toward the narrowed portion 36.

The narrowed portion 36 may be disposed on the ventral side relative to a transverse center line CL1 passing through the center in the longitudinal direction Y of the absorbent main body 2 and on the dorsal side relative to the middle line CL2 between the transverse center line CL1 and the ventral-side edge 2F. The transverse center line CL1 is a portion where a fold is formed when the diaper 1 is folded in two in the longitudinal direction Y. The narrowed portion 36 is disposed near the crotch of the wearer when the diaper 1 is put on the wearer.

As shown in FIG. 1, a pair of channel portions 70 may be formed in the absorbent core 30. The pair of channel portions 70 are disposed symmetrically with respect to the longitudinal center line CW of the absorbent main body 2. The basis weight of the pair of channel portions 70 is set lower than the basis weight of the other portions of the absorbent core 30. Therefore, the density of the pulp fiber and the superabsorbent polymer present in the pair of channel portions 70 is lower than the density of the pulp fiber and the superabsorbent polymer present in the other portions of the absorbent core 30. In an embodiment, the pair of channel portions 70 may be slits having a basis weight of 0.

The pair of channel portions 70 extend in straight lines in the longitudinal direction Y. The pair of channel portions 70 may extend in a curved manner in the longitudinal direction Y. In the diaper 1 shown in FIG. 1, the pair of channel portions 70 are disposed on the ventral side relative to a guide mark 60 described later. That is, the pair of channel portions 70 are formed in positions not overlapping with the guide mark 60 as viewed from the thickness direction Z.

Since the basis weight of the pair of channel portions 70 is lower than the basis weight of the other portions of the absorbent core 30, the rigidity of the pair of channel portions 70 is lower than that of the other portions of the absorbent core 30. Therefore, when the wearer puts on the diaper 1, if the absorbent core 30 comes into contact with the legs of the wearer, the absorbent core 30 is easily bent from the pair of channel portions 70 as starting points.

On the surface of the absorbent core 30, an embossed portion having an uneven shape may be formed by embossing. The embossed portion may be formed in the entire region of the absorbent core 30, or may be formed only in part of the absorbent core 30.

In the absorbent main body 2, a guide mark 60 providing guidance to the position where the buttocks of the wearer are to be disposed is formed. The guide mark 60 is printed on the absorbent main body 2 in such a manner as to be visually recognizable from the skin surface side Z1. As shown in FIG. 1, the guide mark 60 has a substantially W-shaped form imitating the buttocks in order to cause the user to recognize that the guide mark 60 is the position where the buttocks of the wearer are to be disposed.

As shown in FIG. 3, the guide mark 60 is printed on a sheet material 55 disposed between the top sheet 10 and the absorbent core 30. The sheet material 55 is, for example, tissue containing pulp. The surface on the skin surface side Z1 of the sheet material 55 is bonded to the center sheet 11 of the top sheet 10, and the surface on the non-skin surface side Z2 of the sheet material 55 is bonded to the core wrap 38 of the absorbent core 30. A hot melt adhesive, for example, is used for bonding of the sheet material 55. Also a configuration in which only one of the surfaces on the skin surface side Z1 and the non-skin surface side Z2 of the sheet material 55 is bonded to the top sheet 10 or the absorbent core 30 is possible.

The guide mark 60 may be printed on the surface on the non-skin surface side Z2 (the absorbent core 30 side) of the sheet material 55. The guide mark 60 printed on the non-skin surface side Z2 of the sheet material 55 is visually recognizable from the skin surface side Z1 through the center sheet 11 and the sheet material 55. In order to prevent the guide mark 60 from being mistaken for blood or feces, the guide mark may be printed with ink of a color other than red or yellow. For example, the guide mark 60 is printed in blue or green. More specifically, the guide mark 60 is printed in, for example, a color included in the range of 5GY to 10RP of the Munsell hue circle.

As shown in FIG. 1, the guide mark 60 is formed on the dorsal side relative to the narrowed portion 36. In other words, the absorbent core 30 is constricted on the inside in the lateral direction X in a position on the ventral side relative to the guide mark 60. More specifically, the guide mark 60 is disposed on the dorsal side relative to the transverse center line CL1 of the absorbent main body 2, and is disposed on the ventral side relative to the middle line CL3 between the transverse center line CL1 and the end 84F on the ventral side of the dorsal-side fixed portion 84. When viewed from the skin surface side Z1, the guide mark 60 overlaps with the constricted region 43 of the absorbent core 30. The area of the portion on the ventral side relative to the guide mark 60 of the constricted region 43 may be larger than the area of the portion on the dorsal side relative to the guide mark 60 of the constricted region 43.

The guide mark 60 may be formed in a position overlapping with the embossed portion of the absorbent core 30 as viewed from the skin surface side Z1. Wrinkles are less likely to occur in the top sheet 10 disposed on the embossed portion, and therefore the visibility of the guide mark 60 is improved by placing the guide mark 60 in a position overlapping with the embossed portion.

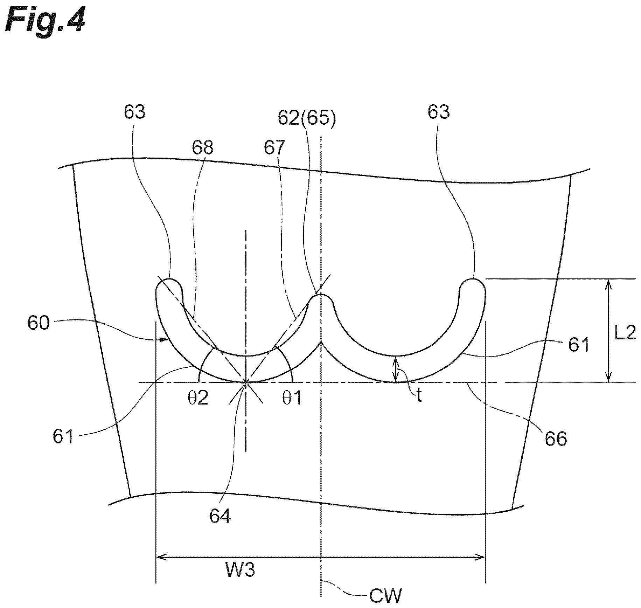

FIG. 4 is an enlarged view of an area around the guide mark 60. As shown in FIG. 4, the guide mark 60 includes two curved portions 61 that are smoothly curved to be convex on the ventral side. Each of the two curved portions 61 has one end 62 and another end 63, and extends in a substantially semicircular arc-shaped form between the one end 62 and the other end 63. The one ends 62 of the two curved portions 61 are coupled to each other in a coupling portion 65. The coupling portion 65 is disposed on the longitudinal center line CW of the absorbent main body 2. The other ends 63 of the two curved portions 61 are located on the outside in the lateral direction X relative to the coupling portion 65. Further, the other ends 63 of the two curved portions 61 are disposed on the dorsal side relative to the coupling portion 65 in the longitudinal direction Y. By a guide mark 60 having the above-described shape being printed on the absorbent main body 2, the user of the diaper 1 associatively imagines the shape of the buttocks from the shape of the guide mark 60, and recognizes that the buttocks of the wearer should be disposed in accordance with the guide mark 60.

The guide mark 60 has a width W3 in the lateral direction X and a width L2 in the longitudinal direction Y. The width W3 in the lateral direction X of the guide mark 60 corresponds to the distance in the lateral direction X between the other ends 63 of the two curved portions 61. When a portion most protruding on the ventral side of the two curved portions 61 is defined as a lowest point 64, the width L2 in the longitudinal direction Y of the guide mark 60 corresponds to the distance in the longitudinal direction Y between the lowest point 64 and the other ends 63 of the two curved portions 61. The width W3 in the lateral direction X of the guide mark 60 is larger than the width L2 in the longitudinal direction Y of the guide mark 60. Thus, the guide mark 60 has a laterally long shape that is long in the lateral direction X.

The width W3 in the lateral direction X of the guide mark 60 is larger than the distance W4 between the pair of channel portions 70 in the lateral direction X. Further, the width W3 in the lateral direction X of the guide mark 60 is smaller than the distance d between the pair of leakage-preventing gathers 80 in the non-standing state. Therefore, as shown in FIG. 1, when the pair of leakage-preventing gathers 80 are in the non-standing state, the coupling portion 65 of the guide mark 60 is exposed to the skin surface side Z1, and the other ends 63 of the two curved portions 61 of the guide mark 60 are covered with the pair of leakage-preventing gathers 80. That is, when the pair of leakage-preventing gathers 80 are in the non-standing state, the guide mark 60 is partially visually recognizable. On the other hand, when the pair of leakage-preventing gathers 80 are in the standing state, the entire guide mark 60, including the other ends 63 of the two curved portions 61 of the guide mark 60, is exposed to the skin surface side Z1.

As shown in FIG. 4, a first angle θ1 that an imaginary line 67 connecting the lowest point 64 and one end 62 of the two curved portions 61 forms with respect to a reference line 66 parallel to the lateral direction X may be larger than a second angle θ2 that an imaginary line 68 connecting the lowest point 64 and another end 63 of the two curved portions 61 forms with respect to the reference line 66. By setting the first angle θ1 larger than the second angle, the guide mark 60 can be formed in a shape imitating the buttocks. In particular, by increasing the first angle θ1, the angle formed by the two curved portions 61 in the coupling portion 65 is reduced, and the coupling portion 65 forms a shape sharply protruding on the dorsal side. By using such a shape, guidance can be made such that the cleft of the buttocks of the wearer will be disposed in agreement with the coupling portion 65.

The two curved portions 61 have a substantially fixed line width t from one ends 62 to the other ends 63. The line width t of the two curved portions 61 may be 0.3% or more and 3.5% or less with respect to the total length L of the absorbent main body 2 in the longitudinal direction Y. By setting the line width t to 0.3% or more and 3.5% or less with respect to the total length L of the absorbent main body 2, displacement of the position of the buttocks can be suppressed while the guide mark 60 is made easily recognizable by the user.

A large number of openings may be formed in the center sheet 11 of the top sheet 10. By a large number of openings being formed in the center sheet 11, the guide mark 60 can be visually recognized from the skin surface side Z1 via the openings, and the guide mark 60 is made easily recognizable. The distance between openings of the top sheet 10 may be smaller than the line width t of the guide mark 60. Thereby, a plurality of openings overlap with the guide mark 60, and thus the visibility of the guide mark 60 can be improved.

In an embodiment, as shown in FIG. 2, the absorbent main body 2 may have an indicator 72 that changes color by contact with moisture. The indicator 72 is provided on the back sheet 20 in such a manner as to be visually recognizable from the non-skin surface side Z2 of the absorbent main body 2, and changes color by reacting with a body fluid absorbed by the absorbent core 30. By the indicator 72 changing color, the user can grasp the wetting state of the absorbent core 30.

As shown in FIG. 2, the indicator 72 includes a center indicator 73 disposed at the center in the lateral direction X of the absorbent main body 2 and a pair of side indicators 74 disposed on the outside in the lateral direction X of the center indicator 73. The center indicator 73 and the pair of side indicators 74 extend in straight lines in the longitudinal direction Y. The center indicator 73 and the pair of side indicators 74 may extend in a wavy or curved manner in the longitudinal direction Y.

The end on the ventral side of the center indicator 73 is disposed on the ventral side relative to the guide mark 60. The end on the dorsal side of the center indicator 73 is disposed on the dorsal side relative to the guide mark 60. That is, the center indicator 73 extends in the longitudinal direction Y to longitudinally cut the guide mark 60. The ends on the dorsal side of the pair of side indicators 74 are disposed on the ventral side relative to the guide mark 60. Thus, the pair of side indicators 74 are formed in positions not overlapping with the guide mark 60 as viewed from the thickness direction Z. Thereby, when the diaper 1 is put on while the buttocks of the wearer are placed in accordance with the guide mark 60, the ends on the dorsal side of the side indicators 74 can be visually recognized. By visually recognizing the ends on the dorsal side of the side indicators 74, it can be checked that the diaper 1 is appropriately put on.

The distance between the pair of side indicators 74 in the lateral direction X may be smaller than the width W3 of the guide mark 60 in the lateral direction X. Thereby, the pair of side indicators 74 are to be disposed on a crotch portion of the wearer, and thus the pair of side indicators 74 easily come into contact with urine during urination. Therefore, the wetting state of the absorbent core 30 can be easily grasped.

As above, the guide mark 60 is disposed on the dorsal side relative to the ends on the dorsal side of the pair of side indicators 74. Also a configuration in which the end on the dorsal side of the center indicator 73 is disposed on the ventral side relative to the guide mark 60 and all of the center indicator 73 and the pair of side indicators 74 are formed on the ventral side relative to the guide mark 60 is possible.

The indicator 72 may include a message indicator 75 that, upon contact with moisture, changes color and displays message information such as text or illustration. The message indicator 75 is disposed on the ventral side of the center indicator 73. A slight gap may be formed between the end on the ventral side of the center indicator 73 and the message indicator 75. As shown in FIG. 2, the distance D1 in the longitudinal direction Y from the transverse center line CL1 to the end on the ventral side of the message indicator 75 may be longer than the distance D2 in the longitudinal direction Y from the transverse center line CL1 to the lowest point 64 of the guide mark 60. Further, the distance D1 in the longitudinal direction Y from the transverse center line CL1 to the end on the ventral side of the message indicator 75 may be longer than the distance D3 in the longitudinal direction Y from the transverse center line CL1 to the other ends 63 of the guide mark 60. A portion on the ventral side relative to the lowest point 64 of the guide mark 60 is deformed when the wearer closes the legs. By increasing the distance D1 in the longitudinal direction Y from the transverse center line CL1 to the end on the ventral side of the message indicator 75, a situation where at the time of exchange of diapers 1 the message indicator 75 is deformed and the visibility of the message indicator 75 is reduced is suppressed.

As shown in FIG. 1, the diaper 1 may further include elastic members 40 that can extend and contract in the length direction. The elastic member 40 is disposed on the outside in the lateral direction X relative to the elastic member 13. The end on the dorsal side of the elastic member 40 is disposed in the dorsal-side region 51, and the end on the ventral side of the elastic member 40 is disposed in the ventral-side region 52. The elastic member 40 is formed of, for example, a stretchable material such as rubber or spandex that can contract in the length direction, and is disposed along the side edge 2S of the absorbent main body 2 between the top sheet 10 and the back sheet 20 of the absorbent main body 2. When the diaper 1 is worn, the elastic members 40 contract in the longitudinal direction Y, and thereby the side edges 2S of the absorbent main body 2 fit around the legs of the wearer. That is, the elastic member 40 forms a leg gather that extends and contracts along the periphery of the leg of the wearer. The diaper 1 may include a plurality of elastic members 40. As shown in FIG. 1, the plurality of elastic members 40 are arranged in the lateral direction X on the outside of the elastic members 13, and extend in the longitudinal direction Y in parallel to each other.

As shown in FIG. 2, the guide mark 60 may be disposed in a position where an imaginary line 60L connecting the lowest point 64 of the guide mark 60 and the edge on the ventral side of a fastening tab 90 (more specifically, as viewed from the thickness direction Z, a position where a side edge of the pair of flap portions 15 and an edge on the ventral side of the pair of fastening tabs 90 intersect each other) intersects an elastic member 40. In this case, the force of pulling the fastening tabs 90 at the time of putting on the diaper 1 is transmitted to the elastic members 40, and fitting condition around the legs is improved.

In the case where the diaper 1 includes a plurality of elastic members 40, the midpoint 60C between the lowest point 64 of the guide mark 60 and the edge on the ventral side of a fastening tab 90 may be disposed on the inside in the lateral direction X relative to an elastic member 40 disposed on the outermost side among the plurality of elastic members 40 (hereinafter, referred to as an "outer elastic member"). The midpoint 60C may be disposed on the inside in the lateral direction X relative to the middle line between an elastic member 40 disposed on the innermost side among the plurality of elastic members 40 (hereinafter, referred to as an "inner elastic member") and the outer elastic member. In the lateral direction X, the midpoint 60C may be disposed on the inside relative to the middle line between the inner elastic member and the outer elastic member and on the outside relative to the inner elastic member.

By placing the guide mark 60 in a position like the above, the tension applied to the fastening tabs 90 at the time of putting on the diaper 1 is transmitted to the elastic members 40, and fitting condition around the legs is improved. In particular, by setting the contraction force of the outer elastic member lower than the contraction force of the inner elastic member, the tension applied to the fastening tab 90 easily acts on the outer elastic member. The "contraction force being low" means that the extension ratio is low. The extension ratio of the elastic member 40 means a ratio S1/S2 between, for a test piece including one elastic member 40 cut out together with the absorbent main body 2, the length S1 of the elastic member 40 when the absorbent main body 2 is pulled until the gather obtained by contraction of the elastic member 40 disappears and the natural length S2 of the elastic member 40 extracted from the test piece.

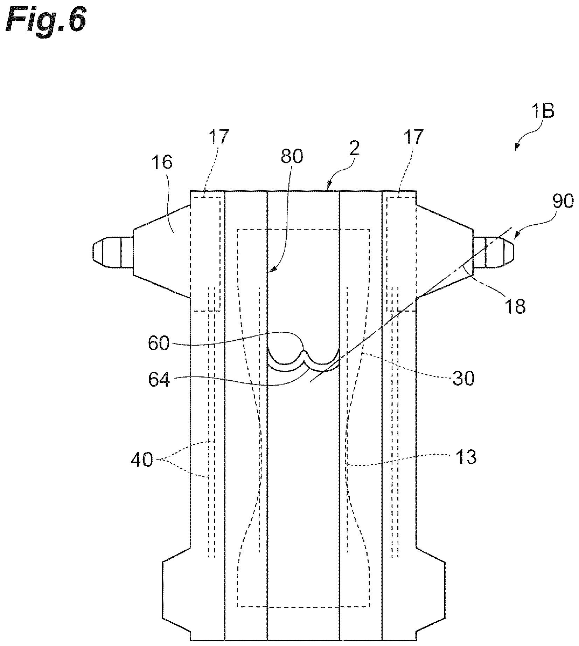

In an embodiment, the pair of flap portions 15 may be stretchable flaps separate from the absorbent main body 2. FIG. 6 is a plan view of a diaper 1B including a pair of stretchable flaps 16 as viewed from the skin surface side Z1. The absorbent main body 2 of the diaper 1B has a substantially rectangular planar shape. Each of the pair of stretchable flaps 16 has a substantially trapezoidal planar shape, and is joined to the absorbent main body 2 via a joint portion 17. The joint portion 17 is a portion where a stacked unit of the stretchable flap 16 and the absorbent main body 2 is joined by a known means such as a hot melt adhesive (HMA), sonic processing, or embossing. When the joint portion 17 is formed using sonic processing or embossing in which fibers are deformed to integrate the stretchable flap 16 and the absorbent main body 2, the rigidity of the joint portion 17 is enhanced, and twisting of the diaper 1B during wearing can be suppressed.

As shown in FIG. 6, when a straight line connecting the end on the ventral side of a joint portion 17 and the lowest point 64 of the guide mark 60 is defined as an imaginary line 18, the imaginary line 18 partially overlaps with a stretchable flap 16 as viewed from the skin surface side Z1. More specifically, as viewed from the skin surface side Z1, the imaginary line 18 intersects a boundary line between the absorbent main body 2 and the pair of stretchable flaps 16. When viewed from the skin surface side Z1, the imaginary line 18 may overlap with a fastening tab 90.

By placing the joint portions 17 and the guide mark 60 in the above-described positional relationship, when putting on the diaper 1, pulling force works from the position of the buttocks of the wearer as a starting point, and thereby the fastening tabs 90 can be fastened without the stretchable flaps being loosened. Therefore, the diaper 1 can be fitted to the wearer.

The imaginary line 18 may intersect an elastic member 40 as viewed from the skin surface side Z1. In this case, the tension acting on the fastening tabs 90 is easily transmitted to the elastic members 40, and fitting condition around the legs is improved.

When putting on the diaper 1 described above, the user places the buttocks of the wearer in accordance with the guide mark 60. More specifically, in a state where the wearer is laid on his/her back, the buttocks of the wearer are disposed in accordance with the two curved portions 61 such that the cleft of the buttocks is positioned on the coupling portion 65. Next, the ventral-side region 52 of the absorbent main body 2 is pulled up to the ventral side of the wearer through the crotch of the wearer. Then, in a state where the ventral-side region 52 of the absorbent main body 2 is kept in contact with the abdomen of the wearer, the pair of fastening tabs 90 attached to the dorsal-side region 51 of the absorbent main body 2 are pulled to the ventral side of the wearer in the waistline direction, and the pair of fastening tabs 90 are fastened to the outer surface of the target portion 45. Thereby, the diaper 1 is put on to cover the abdomen, the back, and the crotch of the wearer. In the diaper 1 described above, since a narrowed portion 36 constricted on the inside in the lateral direction X is formed on the ventral side of the guide mark 60, when pulling up the ventral-side region 52 of the absorbent main body 2 to the ventral side of the wearer, the absorbent core 30 is less likely to interfere with the legs of the wearer. Therefore, a situation where pulling-up of the diaper 1 is insufficient and displacement occurs in the diaper 1 is suppressed. Furthermore, even when the absorbent core 30 comes into contact with the legs of the wearer, the absorbent core 30 is bent from the pair of channel portions 70 having low rigidity as starting points, and therefore insufficient pulling-up of the diaper 1 is more reliably suppressed.

Meanwhile, the tape-type diaper 1 is usually distributed in a state of being housed in a package in a folded state. For example, the diaper 1 is housed in a package in a state where the pair of side sheets 12 are folded on the inside in the lateral direction X along the lateral fixed units 82 and then the diaper 1 is folded in two in the longitudinal direction Y such that the ventral-side region 52 of the absorbent main body overlaps with the dorsal-side region 51. When using the diaper **1,** the user takes out the diaper 1 folded as described above from the package, unfolds the absorbent main body 2 in the longitudinal direction Y, and then spreads the pair of side sheets 12 in the lateral direction X. Here, in the diaper 1, in a state where the pair of side sheets 12 are folded on the inside in the lateral direction X, at least part of the guide mark 60 is formed in a position visually recognizable from the skin surface side Z1. By virtue of the fact that the guide mark 60 can be visually recognized in a state where the pair of side sheets 12 are folded, the presence of the guide mark 60 can reliably be recognized by the user.

Although the diaper 1 shown in FIG. 1 includes an absorbent core 30 having a pair of channel portions 70, it is not always necessary that a pair of channel portions 70 be formed in the absorbent core 30. Even in the case where a pair of channel portions 70 are not formed, as long as a narrowed portion 36 in which the width in the lateral direction X is partially constricted is formed in the absorbent core 30 on the ventral side of the guide mark 60, the absorbent core 30 is less likely to interfere with the legs of the wearer, and therefore displacement of the diaper 1 can be suppressed.

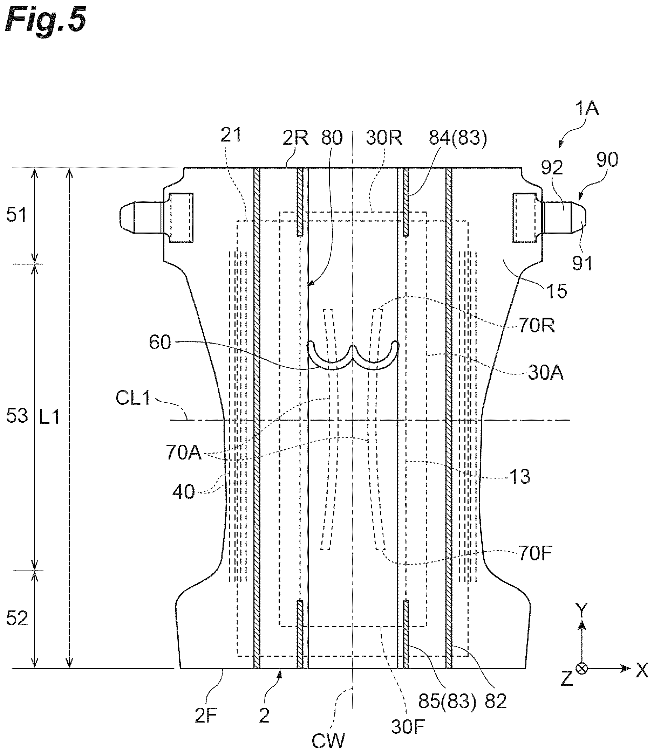

As long as a pair of channel portions 70 are formed in the absorbent core 30, the absorbent core 30 may not be constricted in a position on the ventral side relative to the guide mark 60. A diaper 1A according to another embodiment will now be described with reference to FIG. 5. In the following, differences of the diaper 1A shown in FIG. 1 are mainly described, and a repeated description is omitted.

The diaper 1A shown in FIG. 5 includes an absorbent core 30A in place of the absorbent core 30. The absorbent core 30A is different from the absorbent core 30 in that the absorbent core 30A has a substantially fixed width in the entire region in the longitudinal direction Y. A pair of channel portions 70A are formed in the absorbent core 30A. The pair of channel portions 70A are disposed symmetrically with respect to the longitudinal center line CW of the absorbent main body 2. The basis weight of the pair of channel portions 70A is set lower than the basis weight of the other portions of the absorbent core 30A. In an embodiment, the pair of channel portions 70A may be slits having a basis weight of 0.

The pair of channel portions 70A extend in a curved manner in the longitudinal direction Y. The pair of channel portions 70A may extend in straight lines in the longitudinal direction Y. The pair of channel portions 70 extend to longitudinally cut the guide mark 60. More specifically, the ends 70R on the dorsal side of the pair of channel portions 70A are disposed on the dorsal side relative to the guide mark 60, and the ends 70F on the ventral side of the pair of channel portions 70A are disposed on the ventral side relative to the guide mark 60. Thus, parts of the pair of channel portions 70 are formed on the ventral side of the guide mark 60.

Here, the length of the portion on the ventral side relative to the guide mark 60 of each of the pair of channel portions 70A is longer than the length of the portion on the dorsal side relative to the guide mark 60 of each of the pair of channel portions 70A. That is, large portions of the pair of channel portions 70 are disposed on the ventral side relative to the guide mark 60. In the diaper 1A described above, since a pair of channel portions 70A are formed on the ventral side of the guide mark 60, when pulling up the ventral-side region 52 of the absorbent main body 2 to the ventral side of the wearer, the absorbent core 30 is easily bent from the pair of channel portions 70A as starting points. Therefore, the absorbent core 30 is less likely to interfere with the legs of the wearer, and insufficient pulling-up of the diaper 1 is suppressed.

Hereinabove, diapers according to various embodiments are described; however, the present invention is not limited to the above-described embodiments, and various modification aspects can be configured without changing the gist of the invention. That is, it should be noted that the above-described embodiments are for the purpose of describing examples and do not limit the scope of the present invention.

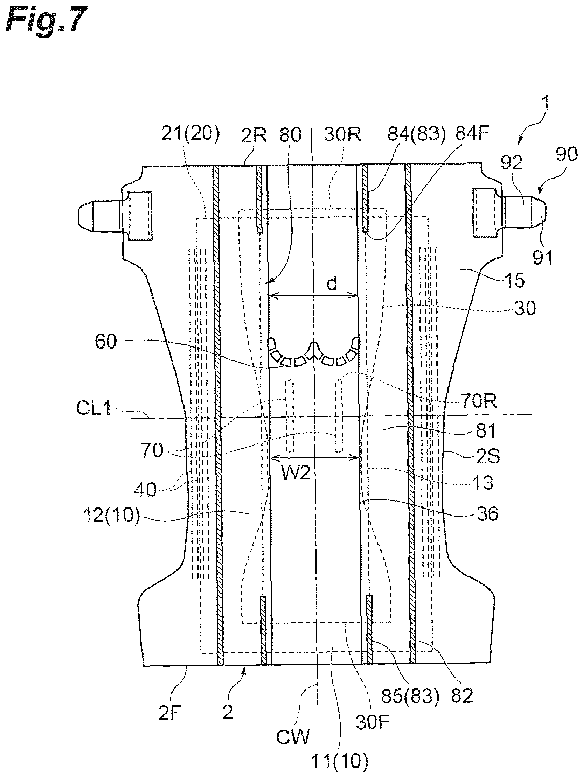

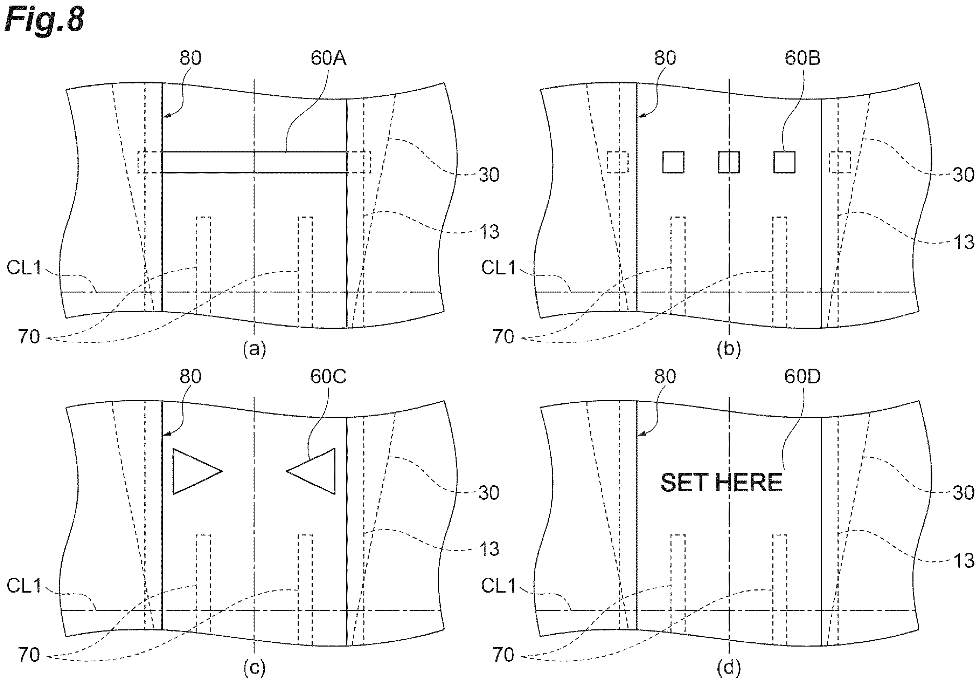

For example, as shown in FIG. 7, the guide mark 60 may not be in a solid line, and may be in a broken line or a dotted line. Further, as long as guidance to the position where the buttocks of the wearer are to be placed can be provided, the shape of the guide mark 60 is not limited to shapes imitating the buttocks. FIGS. 8(a) to 8(d), 9(a) to 9(d), and 10 show modification examples of the guide mark. The modification examples of the guide mark will now be described.

For example, a guide mark 60A shown in FIG. 8(a) extends in a straight line in the lateral direction X. The length of the guide mark 60A in the lateral direction X may be longer than the distance between the pair of leakage-preventing gathers 80. A guide mark 60B shown in FIG. 8(b) includes a plurality of quadrangular figures arranged with spaces therebetween in the lateral direction X, and is formed in a broken line as a whole. A guide mark 60C shown in FIG. 8(c) includes a pair of triangular figures facing each other in the lateral direction X. The pair of triangular figures are formed line-symmetrically with respect to the longitudinal center line CW, and one of the vertexes of each figure is oriented in the lateral direction X in order to indicate the position where the buttocks are to be placed. A guide mark 60D shown in FIG. 8(d) includes characters providing guidance to the position where the buttocks are to be placed.

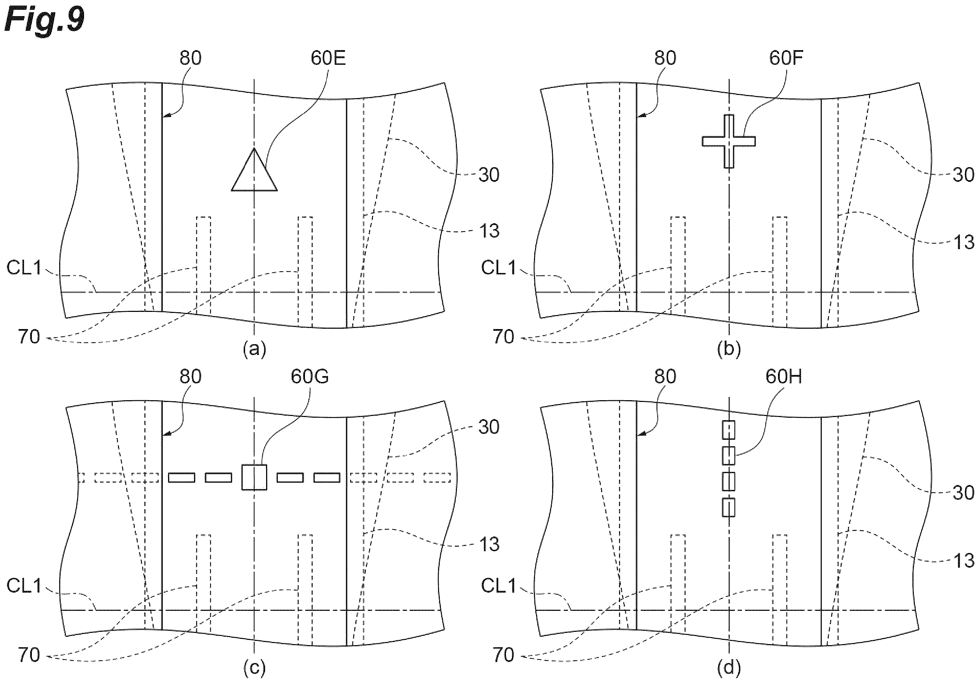

A guide mark 60E shown in FIG. 9(a) includes a triangular figure that is disposed on the longitudinal center line CW and of which one vertex is oriented to the dorsal side. The one vertex indicates the position where the cleft of the buttocks is to be placed. A guide mark 60F shown in FIG. 9(b) includes a cross-shaped figure disposed on the longitudinal center line CW. The intersection position of this figure indicates the position where the cleft of the buttocks of the wearer is to be placed. A guide mark 60G shown in FIG. 9(c) includes a quadrangular figure disposed on the longitudinal center line CW and a broken line formed along the lateral direction X to intersect the figure. A guide mark 60H shown in FIG. 9(d) includes a broken line disposed on the longitudinal center line CW and formed along the longitudinal direction Y.

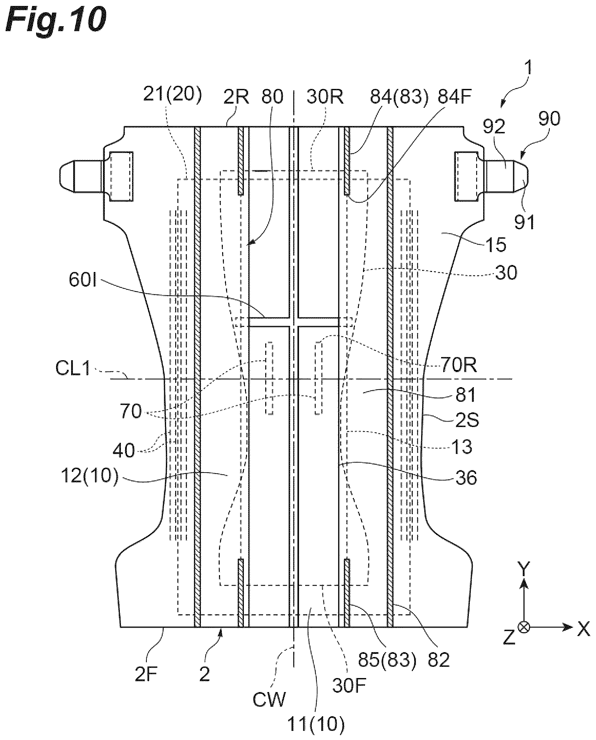

A guide mark 60I shown in FIG. 10 includes a straight line extending in the lateral direction X and a straight line extending in the longitudinal direction Y. The length of the straight line extending in the lateral direction X is longer than the distance between the pair of leakage-preventing gathers 80 and shorter than the total width of the absorbent main body 2 in the lateral direction X. The straight line extending in the longitudinal direction Y extends over the total length of the absorbent main body 2 in the longitudinal direction Y. The intersection position of the straight line extending in the lateral direction X and the straight line extending in the longitudinal direction Y indicates the position where the cleft of the buttocks of the wearer is to be placed.

The guide mark 60 may be a figure such as an illustration of an animal or the like or a heart mark. The various embodiments described above can be combined to the extent that there is no contradiction.

The present invention will now be described with reference to the following enumerated clauses. Even though there is no specific enumeration, the present invention may include arbitrary combinations of the following clauses.
1. A diaper extending in a longitudinal direction connecting from a ventral side to a dorsal side of a wearer and in a lateral direction perpendicular to the longitudinal direction,
   the diaper comprising:
   an absorbent main body including a top sheet disposed on a skin surface side of the wearer, a back sheet disposed on a non-skin surface side opposed to the skin surface side, and an absorbent core disposed between the top sheet and the back sheet; and
   a pair of fastening tabs extending out from the absorbent main body to an outside in the lateral direction,
   wherein the absorbent main body is formed with a guide mark for guiding a position where buttocks of the wearer are to be placed so as to be visible from the skin surface side, and
   the absorbent core is at least partially constricted inward in the lateral direction at a position on the ventral side relative to the guide mark.
2. The diaper according to clause 1, in which
   the absorbent core has a pair of channel portions formed symmetrically with respect to a longitudinal center line passing through a center in the lateral direction of the absorbent main body and having a lower basis weight than other portions of the absorbent core, and
   at least parts of the pair of channel portions are disposed on the ventral side relative to the guide mark.
3. The diaper according to clause 1 or 2, in which the absorbent core includes a constricted region having a narrower width in the lateral direction than an end portion on the dorsal side of the absorbent core, and
   as viewed from the skin surface side, the guide mark is disposed in a position overlapping with the constricted region.
4. The diaper according to any one of clauses 1 to 3, in which an area of a portion of the constricted region on the ventral side relative to the guide mark is larger than an area of a portion of the constricted region on the dorsal side relative to the guide mark.
5. The diaper according to any one of clauses 1 to 4, in which the absorbent main body has an indicator extending in the longitudinal direction and configured to change color by reacting with a body fluid of the wearer, and
   the guide mark is disposed on the dorsal side relative to an end on the dorsal side of the indicator.
6. The diaper according to any one of clauses 1 to 5, in which the top sheet includes a center sheet covering the absorbent core and a pair of side sheets covering both side portions of the center sheet in the lateral direction,
   each of the pair of side sheets includes a dorsal-side fixed portion fixed to the center sheet on the dorsal side relative to a transverse center line passing through a center in the longitudinal direction of the absorbent main body, and
   in the longitudinal direction, the guide mark is disposed on the ventral side relative to a middle line between an end on the ventral side of the dorsal-side fixed portion and the transverse center line.
7. The diaper according to any one of clauses 1 to 6, in which the guide mark has a shape imitating a shape of buttocks.
8. The diaper according to any one of clauses 1 to 7, in which the guide mark includes two curved portions convex on the ventral side.
9. The diaper according to any one of clauses 1 to 8, in which the guide mark includes a coupling portion in which one ends of the two curved portions are coupled to each other, and
   the coupling portion is placed on a longitudinal center line passing through a center in the lateral direction of the absorbent main body.
10. The diaper according to any one of clauses 1 to 9, in which other ends of the two curved portions are disposed on the dorsal side relative to the coupling portion.
11. The diaper according to any one of clauses 1 to 10, in which when a portion most protruding on the ventral side of the two curved portions is defined as a lowest point, a first angle formed by an imaginary line connecting the lowest point and one end of the two curved portions with respect to a reference line parallel to the lateral direction is larger than a second angle formed by an imaginary line connecting the lowest point and the other end of the two curved portions with respect to the reference line.
12. The diaper according to any one of clauses 1 to 11, in which a line width of the two curved portions is 0.3% or more and 3.5% or less with respect to a total length of the absorbent main body in the longitudinal direction.
13. The diaper according to any one of clauses 1 to 12, in which a width of the guide mark in the lateral direction is larger than a width of the guide mark in the longitudinal direction.
14. The diaper according to any one of clauses 1 to 13, in which the absorbent main body includes a pair of standing leakage-preventing gathers and placed on an outside in the lateral direction of the absorbent core, and
   when the pair of leakage-preventing gathers are in a standing state, other ends of the two curved portions of the guide mark are exposed, and when the pair of leakage-preventing gathers are in a non-standing state, the other ends of the two curved portions of the guide mark are covered with the pair of leakage-preventing gathers.
15. The diaper according to any one of clauses 1 to 14, in which the pair of channel portions extend from a position on the ventral side relative to the guide mark to a position on the dorsal side relative to the guide mark, and
   a length of a portion of the pair of channel portions on the ventral side relative to the guide mark is longer than a length of a portion of the pair of channel portions on the dorsal side relative to the guide mark.
16. The diaper according to any one of clauses 1 to 15, in which a width of the guide mark in the lateral direction is larger than a distance between the pair of channel portions in the lateral direction.
17. The diaper according to any one of clauses 1 to 16, in which ends on the dorsal side of the pair of channel portions are disposed on the ventral side relative to the guide mark.
18. The diaper according to any one of clauses 1 to 17, in which the absorbent core includes an embossed portion subjected to unevenness processing, and
   as viewed from the skin surface side, the guide mark is disposed at a position overlapping with the embossed portion.
19. The diaper according to any one of clauses 1 to 18, in which the guide mark is printed on a sheet material disposed between the top sheet and the absorbent core.
20. The diaper according to any one of clauses 1 to 19, in which the sheet material is tissue containing pulp.
21. The diaper according to any one of clauses 1 to 20, in which the guide mark is printed on a surface on the non-skin surface side of the sheet material.
22. The diaper according to any one of clauses 1 to 21, in which each of a surface on the skin surface side and a surface on the non-skin surface side of the sheet material is bonded to the top sheet or the absorbent core with a hot melt adhesive.
23. The diaper according to any one of clauses 1 to 22, in which when the side sheets are folded on an inside in the lateral direction, at least part of the guide mark is formed in a position visually recognizable from the skin surface side.
24. The diaper according to clause 1, in which the guide mark is printed in a color included in a range of 5GY to 10RP of a Munsell hue circle.
25. A diaper extending in a longitudinal direction connecting from a ventral side to a dorsal side of a wearer and in a lateral direction perpendicular to the longitudinal direction,
   the diaper comprising:
   an absorbent main body including a top sheet disposed on a skin surface side of the wearer, a back sheet disposed on a non-skin surface side of the wearer, and an absorbent core disposed between the top sheet and the back sheet; and
   a pair of fastening tabs extending out from the absorbent main body to an outside in the lateral direction,
   wherein the absorbent main body is formed with a guide mark for guiding a position where buttocks of the wearer are to be placed so as to be visible from the skin surface side,
   the absorbent core has a pair of channel portions formed symmetrically with respect to a longitudinal center line passing through a center in the lateral direction of the absorbent main body and having a lower basis weight than other portions of the absorbent core, and
   at least parts of the pair of channel portions are disposed on the ventral side relative to the guide mark.
26. The diaper according to clause 25, in which the absorbent core is at least partially constricted inward in the lateral direction at a position on the ventral side relative to the guide mark.
27. A diaper extending in a longitudinal direction connecting from a ventral side to a dorsal side of a wearer and in a lateral direction perpendicular to the longitudinal direction,
   the diaper including:
   an absorbent main body including a top sheet disposed on a skin surface side of the wearer, a back sheet disposed on a non-skin surface side opposed to the skin surface side, and an absorbent core disposed between the top sheet and the back sheet;
   a pair of fastening tabs extending out from the absorbent main body to an outside in the lateral direction; and
   elastic members disposed along both edges in the lateral direction of the absorbent main body and configured to extend and contract in the longitudinal direction,
   in which the absorbent main body is formed with a guide mark for guiding a position where buttocks of the wearer are to be placed so as to be visible from the skin surface side, and
   when viewed from the skin surface side, an imaginary line connecting an edge on the ventral side of the guide mark and an edge on the ventral side of the pair of fastening tabs intersects either of the elastic members.
28. A diaper extending in a longitudinal direction connecting from a ventral side to a dorsal side of a wearer and in a lateral direction perpendicular to the longitudinal direction,
   the diaper including:
   an absorbent main body including a top sheet disposed on a skin surface side of the wearer, a back sheet disposed on a non-skin surface side opposed to the skin surface side, and an absorbent core disposed between the top sheet and the back sheet;
   a pair of stretchable flaps extending out from the absorbent main body to an outside in the lateral direction; and
   a pair of fastening tabs attached to the pair of stretchable flaps,
   in which the absorbent main body is formed with a guide mark for guiding a position where buttocks of the wearer are to be placed so as to be visible from the skin surface side,
   the pair of stretchable flaps are joined to the absorbent main body via joint portions, and
   when viewed from the skin surface side, an imaginary line connecting an edge on the ventral side of the guide mark and an edge on the ventral side of the joint portions partially overlaps with either of the pair of stretchable flaps.
29. A diaper extending in a longitudinal direction connecting from a ventral side to a dorsal side of a wearer and in a lateral direction perpendicular to the longitudinal direction,
   the diaper including:
   an absorbent main body including a top sheet disposed on a skin surface side of the wearer, a back sheet disposed on a non-skin surface side opposed to the skin surface side, and an absorbent core disposed between the top sheet and the back sheet; and
   a pair of fastening tabs extending out from the absorbent main body to an outside in the lateral direction,
   in which the absorbent main body is formed with a guide mark for guiding a position where buttocks of the wearer are to be placed so as to be visible from the skin surface side,
   the absorbent main body has a center indicator extending in the longitudinal direction and configured to change color by reacting with a body fluid of the wearer and a message indicator placed on the ventral side of the center indicator and configured to change color by reacting with a body fluid of the wearer and display message information, and
   in the longitudinal direction, a distance from a transverse center line passing through a center in the longitudinal direction of the absorbent main body to an end on the ventral side of the message indicator is longer than a distance from the transverse center line to the guide mark.

### Reference Signs List

- 1, 1A: diaper
- 10: top sheet
- 11: center sheet
- 12: side sheet
- 20: back sheet
- 30, 30A: absorbent core
- 43: constricted region
- 55: sheet material
- 60: guide mark
- 61: curved portion
- 62: one end
- 63: another end
- 64: lowest point
- 65: coupling portion
- 66: reference line
- 67, 68: imaginary line
- 70, 70A: channel portion
- 72: indicator
- 80: leakage-preventing gather
- 84: dorsal-side fixed portion
- 90: fastening tab
- CL1: transverse center line
- CW: longitudinal center line
- X: lateral direction
- Y: longitudinal direction
- Z1: skin surface side
- Z2: non-skin surface side
- θ1: first angle
- θ2: second angle

## Claims

1. A diaper extending in a longitudinal direction connecting from a ventral side to a dorsal side of a wearer and in a lateral direction perpendicular to the longitudinal direction,
the diaper comprising:
an absorbent main body including a top sheet disposed on a skin surface side of the wearer, a back sheet disposed on a non-skin surface side opposed to the skin surface side, and an absorbent core disposed between the top sheet and the back sheet; and
a pair of fastening tabs extending out from the absorbent main body to an outside in the lateral direction,
wherein the absorbent main body is formed with a guide mark for guiding a position where buttocks of the wearer are to be placed so as to be visible from the skin surface side, and
the absorbent core is at least partially constricted inward in the lateral direction at a position on the ventral side relative to the guide mark.

2. The diaper according to claim 1, wherein the absorbent core has a pair of channel portions formed symmetrically with respect to a longitudinal center line passing through a center in the lateral direction of the absorbent main body and having a lower basis weight than other portions of the absorbent core, and
at least parts of the pair of channel portions are disposed on the ventral side relative to the guide mark.

3. The diaper according to claim 1, wherein the absorbent core includes a constricted region having a narrower width in the lateral direction than an end portion on the dorsal side of the absorbent core, and
as viewed from the skin surface side, the guide mark is disposed at a position overlapping with the constricted region.

4. The diaper according to claim 3, wherein an area of a portion of the constricted region on the ventral side relative to the guide mark is larger than an area of a portion of the constricted region on the dorsal side relative to the guide mark.

5. The diaper according to claim 1, wherein the absorbent main body has an indicator extending in the longitudinal direction and configured to change color by reacting with a body fluid of the wearer, and
the guide mark is disposed on the dorsal side relative to an end on the dorsal side of the indicator.

6. The diaper according to claim 1, wherein the top sheet includes a center sheet covering the absorbent core and a pair of side sheets covering both side portions of the center sheet in the lateral direction,
each of the pair of side sheets includes a dorsal-side fixed portion fixed to the center sheet on the dorsal side relative to a transverse center line passing through a center in the longitudinal direction of the absorbent main body, and
in the longitudinal direction, the guide mark is disposed on the ventral side relative to a middle line between an end on the ventral side of the dorsal-side fixed portion and the transverse center line.

7. The diaper according to claim 1, wherein the guide mark has a shape imitating a shape of buttocks.

8. The diaper according to claim 1, wherein the guide mark includes two curved portions convex on the ventral side.

9. The diaper according to claim 8, wherein the guide mark includes a coupling portion in which one ends of the two curved portions are coupled to each other, and
the coupling portion is placed on a longitudinal center line passing through a center in the lateral direction of the absorbent main body.

10. The diaper according to claim 9, wherein other ends of the two curved portions are disposed on the dorsal side relative to the coupling portion.

11. The diaper according to claim 10, wherein when a portion most protruding on the ventral side of the two curved portions is defined as a lowest point, a first angle formed by an imaginary line connecting the lowest point and one end of the two curved portions with respect to a reference line parallel to the lateral direction is larger than a second angle formed by an imaginary line connecting the lowest point and the other end of the two curved portions with respect to the reference line.

12. The diaper according to claim 8, wherein a line width of the two curved portions is 0.3% or more and 3.5% or less with respect to a total length of the absorbent main body in the longitudinal direction.

13. The diaper according to claim 1, wherein a width of the guide mark in the lateral direction is larger than a width of the guide mark in the longitudinal direction.

14. The diaper according to claim 10, wherein the absorbent main body includes a pair of standing leakage-preventing gathers disposed on an outside in the lateral direction of the absorbent core, and
when the pair of leakage-preventing gathers are in a standing state, other ends of the two curved portions of the guide mark are exposed, and when the pair of leakage-preventing gathers are in a non-standing state, the other ends of the two curved portions of the guide mark are covered with the pair of leakage-preventing gathers.

15. The diaper according to claim 2, wherein the pair of channel portions extend from a position on the ventral side relative to the guide mark to a position on the dorsal side relative to the guide mark, and
a length of a portion of the pair of channel portions on the ventral side relative to the guide mark is longer than a length of a portion of the pair of channel portions on the dorsal side relative to the guide mark.

16. The diaper according to claim 15, wherein a width of the guide mark in the lateral direction is larger than a distance between the pair of channel portions in the lateral direction.

17. The diaper according to claim 2, wherein ends on the dorsal side of the pair of channel portions are disposed on the ventral side relative to the guide mark.

18. The diaper according to claim 1, wherein the absorbent core includes an embossed portion subjected to unevenness processing, and
as viewed from the skin surface side, the guide mark is disposed at a position overlapping with the embossed portion.

19. The diaper according to claim 1, wherein the guide mark is printed on a sheet material disposed between the top sheet and the absorbent core.

20. The diaper according to claim 19, wherein the sheet material is tissue containing pulp.

21. The diaper according to claim 19, wherein the guide mark is printed on a surface on the non-skin surface side of the sheet material.

22. The diaper according to claim 19, wherein each of a surface on the skin surface side and a surface on the non-skin surface side of the sheet material is bonded to the top sheet or the absorbent core with a hot melt adhesive.

23. The diaper according to claim 6, wherein when the side sheets are folded on an inside in the lateral direction, at least part of the guide mark is formed in a position visually recognizable from the skin surface side.

24. The diaper according to claim 1, wherein the guide mark is printed in a color included in a range of 5GY to 10RP of a Munsell hue circle.

25. A diaper extending in a longitudinal direction connecting from a ventral side to a dorsal side of a wearer and in a lateral direction perpendicular to the longitudinal direction,
the diaper comprising:
an absorbent main body including a top sheet disposed on a skin surface side of the wearer, a back sheet disposed on a non-skin surface side of the wearer, and an absorbent core disposed between the top sheet and the back sheet; and
a pair of fastening tabs extending out from the absorbent main body to an outside in the lateral direction,
wherein the absorbent main body is formed with a guide mark for guiding a position where buttocks of the wearer are to be placed so as to be visible from the skin surface side,
the absorbent core has a pair of channel portions formed symmetrically with respect to a longitudinal center line passing through a center in the lateral direction of the absorbent main body and having a lower basis weight than other portions of the absorbent core, and
at least parts of the pair of channel portions are disposed on the ventral side relative to the guide mark.

26. The diaper according to claim 25, wherein the absorbent core is at least partially constricted inward in the lateral direction at a position on the ventral side relative to the guide mark.
